# EUROPEAN PATENT APPLICATION

(11) **EP 0 709 067 A2**
(43) Date of publication of application: **01.05.1996**
(21) Application number: 95307687.4
(22) Date of filing: 27.10.1995
(51) Int. Cl.: A61F 2/06

(54) **Stent fabrication method**

(30) Priority: 27.10.1994 US 330625
(71) Applicant: MEDINOL LIMITED, Tel Aviv 61581 (IL)
(72) Inventor: Pinchasik, Gregory, Ramat Hasharon 47414 (IS); Yaron, Ira, Jerusalem 90836 (IS)
(74) Representative: Bayliss, Geoffrey Cyril

(57) **Abstract**

A stent and a method for fabricating the stent are disclosed. The stent has an originally flat pattern and connection points where the sides of the flat pattern are joined. The method includes the steps of a) cutting (12) a stent pattern into a flat piece of metal thereby to produce a metal pattern, b) deforming (14) the metal pattern so as to cause two opposing sides to meet, and c) joining (16) the two opposing sides at least at one point.

## Description

The present invention relates generally to methods of fabricating stents.

Stents are known in the art. They are typically formed of a cylindrical metal mesh which can expand when pressure is internally applied. Alternatively, they can be formed of wire wrapped into a cylindrical shape.

As described in US 4776337 to Palmaz, the cylindrical metal mesh shape is produced by laser cutting a thin walled metal tube. The laser cuts away all but the lines and curves of the mesh.

The method of US 4776337 is applicable for relatively large mesh shapes and for meshes whose lines are relatively wide. However, for more delicate and/or intricate shapes, the spot size of the laser is too large.

It is therefore, an object of the present invention to provide a stent fabrication method which can produce stents with relatively intricate and/or delicate designs.

The method involves first creating a flat version of the desired stent pattern from a piece of thin sheet metal. The flat pattern can be produced through any suitable technique, such as etching the design into the sheet metal, or by cutting with a very fine laser, shoulder one become commercially available or by any other technique.

Once the sheet metal has been cut, it is deformed so as to cause its edges to meet. To create a cylindrical stent from a flat, roughly rectangular metal pattern, the flat metal is rolled until the edges meet. The locations where edges meet are joined together, such as by spot welding. Afterwards, the stent is polished, either mechanically or electrochemically.

The present invention will be understood and appreciated more fully from the following detailed description taken in conjunction with the drawings in which:
Figure 1 is a flow chart illustration of the stent fabrication method of the present invention;
Figures 2A, 2B and 2C are illustrations of three alternative stent patterns to be etched, in accordance with the method of Figure 1, into a flat sheet of metal;
Figure 3 is an isometric illustration of a stent being deformed, useful in understanding the method of Figure 1;
Figure 4 is an isometric illustration of a stent formed from the method of Figure 1;
Figures 5A and 5B are side and top view illustrations, respectively, of one connection location of the stent of Figure 4; and
Figure 6 is a side view illustration of one connection location of the stent of Figure 4 which is connected in a nail-like manner.

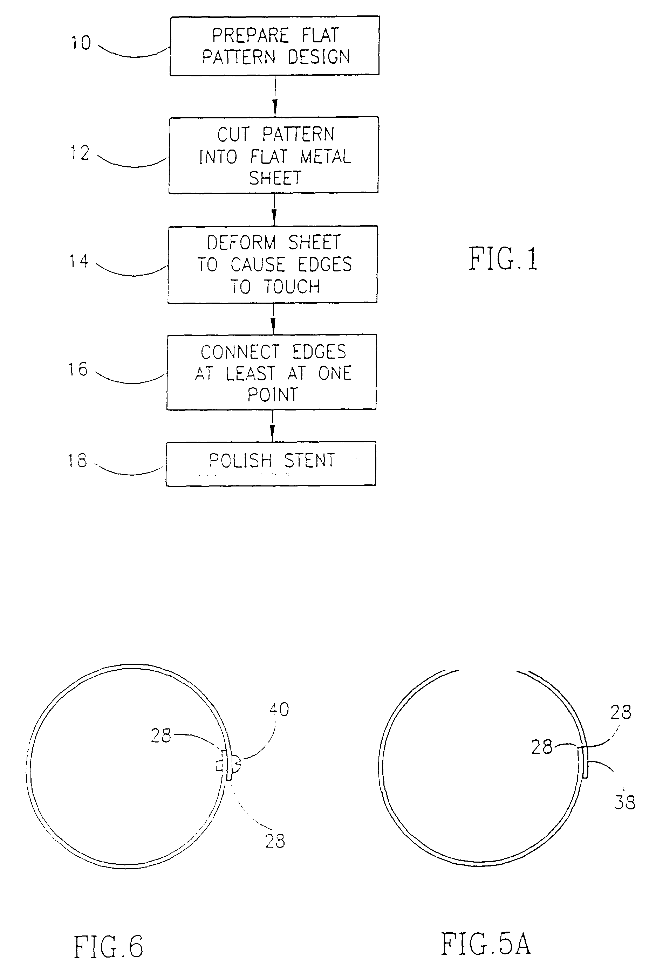

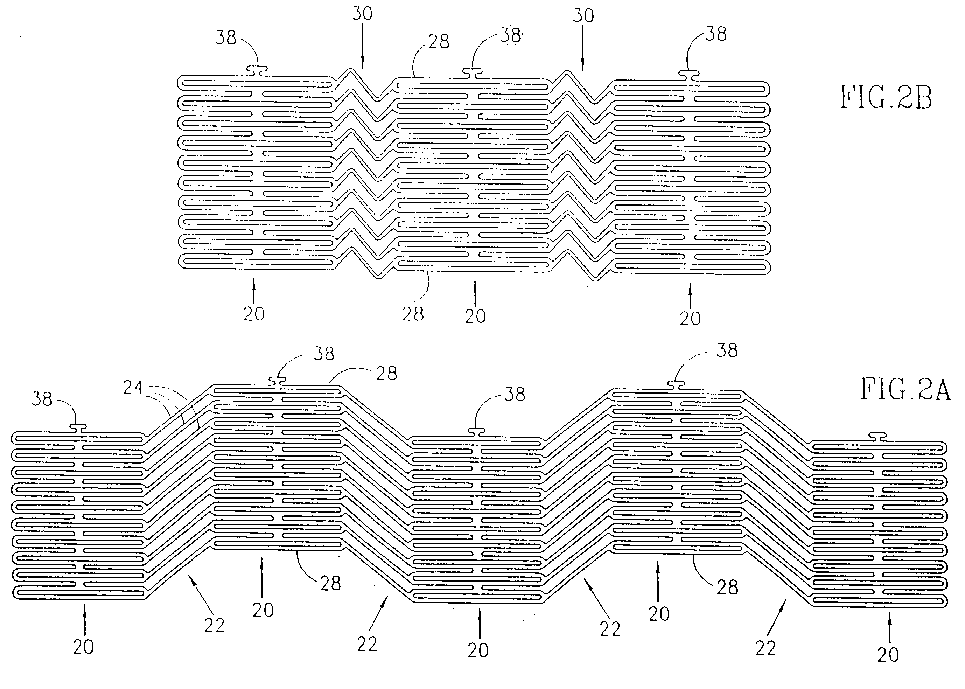

Reference is now made to Figure 1, which illustrates the stent fabrication method of the present invention and to Figures 2A, 2B, 2C, 3 and 4 which are useful in understanding the method of Figure 1.

In the stent fabrication method the present invention, a stent designer first prepares a drawing of the desired stent pattern in a flat format (step 10).

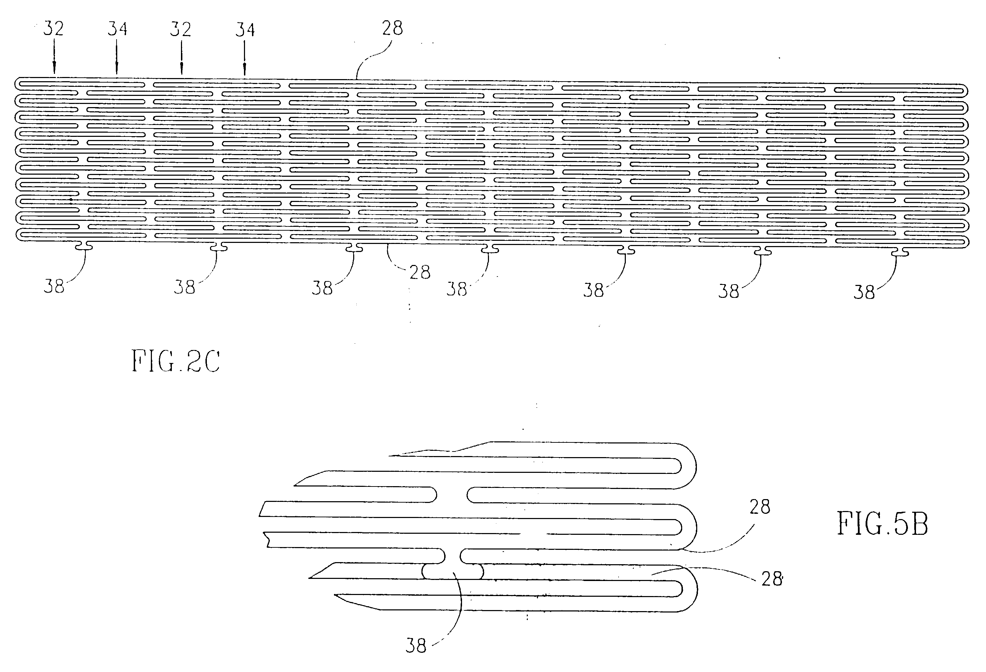

Figures 2A, 2B and 2C illustrate three exemplary stent pattern designs. The pattern of Figure 2A has two types of sections 20 and 22. Each section 20 has two opposing periodic patterns and each section 22 has a plurality of connecting lines 24. The pattern of Figure 2A can be formed of any size; a preferable size is to have each section 20 be between 1 and 6mm wide and each section 22 have connecting lines 24 of 1 - 6mm long. At such sizes, the pattern of Figure 2A cannot be cut using a laser cutting system.

The pattern of Figure 2B is similar to that of Figure 2A in that it also has sections of opposing periodic patterns. The pattern of Figure 2B also has connecting sections, labelled 30, which has a Z shape.

The pattern of Figure 2C has no connecting sections. Instead, it has a series of alternating patterns, labelled 32 and 34.

The patterns of Figures 2A, 2B and 2C optionally also have a plurality of small protrusions 38 which are useful in forming the stent, as described hereinbelow.

Returning to Figure 1, in step 12, the stent pattern is cut into a flat piece of metal ("sheet metal"). The metal can be any type of biocompatible material, such as stainless steel, or a material which is plated with a biocompatible material. The cutting operation can be implemented in any of a number of ways, such as by etching, or by cutting with a fine cutting tool, or by cutting with a very fine laser, should one become commercially available.

If step 12 is implemented with etching, then, the process is designed to cut through the sheet metal. This process is known, however, for the purposes of completeness, it will be briefly described hereinbelow.

The drawing of the pattern is reduced and printed onto a transparent film. Since it is desired to cut completely through the metal, the drawing is printed onto two films which are joined together in a few places along their edges. The sheet metal is covered, on both sides, with a layer of photoresist and placed between the two transparent, printed films. The structure is illuminated on both sides which causes the portions of the photoresist which receive the light (which are all the empty spaces in the pattern, such as spaces 26 of Figure 2A) to change properties.

The sheet metal is placed into acid which eats away those portions of the photoresist which changed properties. The sheet metal is then placed into an etching solution which etches away all material on which there is no photoresist. The resultant sheet metal is placed into a photoresist-removing solution which removes the photoresist, leaving the metal having the desired stent pattern.

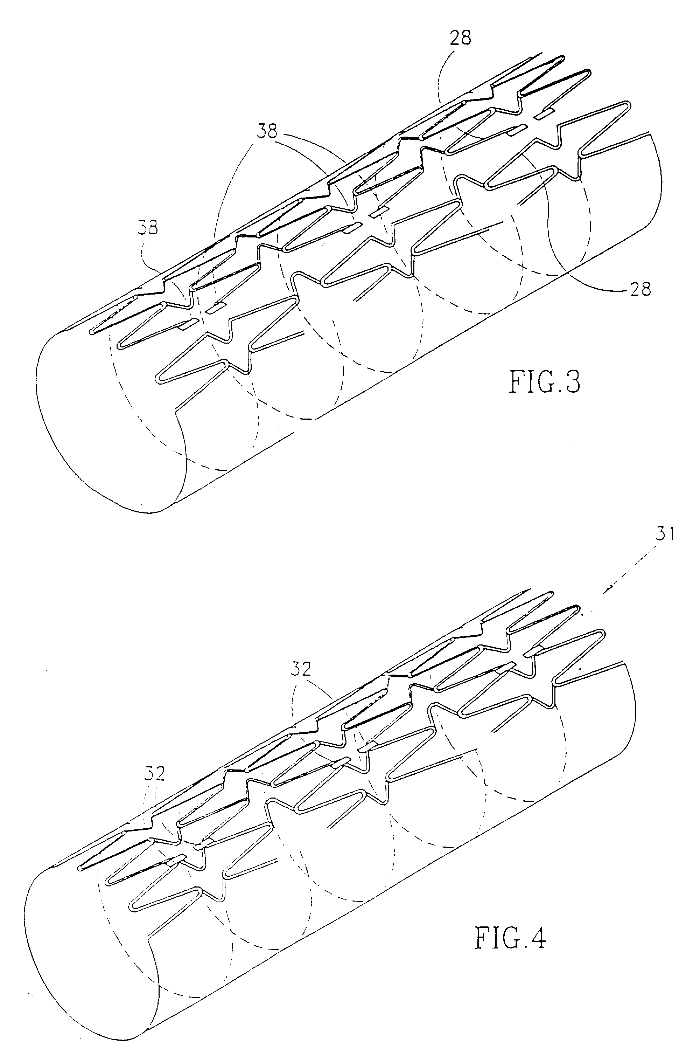

In step 14, the metal pattern is deformed so as to cause its long sides (labelled 23 in Figures 2A, 2B and 2C) to meet each other. Figure 3 illustrates the deformation process. For cylindrical stents, the deformation process is a rolling process, as shown.

If the protrusions 38 have been produced, after deformation of the metal pattern, the protrusions 38 protrude over the edge 28 to which they are not attached. This is illustrated in Figure 5A.

In step 16, the edges 28 are joined together by any suitable process, such as spot welding. If the protrusions 33 were made, the protrusions 38 are joined to the opposite edge 23, either by welding, adhesive or, as illustrated in Figure 6, with a nail-like element 40. Figure 5B illustrates the connection of the protrusion 33 to the opposite edge 23. Since protrusion 38 is typically designed to extend the width of one loop 39, the pattern is approximately preserved. This is seen in Figure 5B.

Alternatively, the edges 28 can be brought together and joined in the appropriate places.

Figure 4 illustrates a stent 31 formed by the process of steps 10-16 for the pattern of Figure 2A. It is noted that such a stent has connection points 32 formed by the joining of the points 30.

Finally, the stent 31 is polished to remove any excess material not properly removed by the cutting process (step 12). The polishing can be performed mechanically, by rubbing a polishing stick having diamond dust on its outside inside the stent 31. Alternatively, an electropolishing unit can be utilised.

It is be appreciated by persons skilled in the art that the present invention is not limited to what has been particularly shown and described hereinabove. Rather the scope of the present invention is defined only by the claims which follow:

## Claims

1. A stent fabrication method comprising the steps of:
cutting a stent pattern into a flat piece of metal thereby to produce a metal pattern;
deforming said metal pattern so as to cause two opposing sides to meet; and
joining said two opposing sides at least at one point thereby to produce a stent having said pattern.

2. A method according to claim 1 and also including the step of polishing said stent.

3. A method according to claim 1 or claim 2, wherein said step of cutting comprises the step of etching said metal to produce said pattern.

4. A stent having an originally flat, stent pattern and connection points where sides of said flat pattern are joined.

5. A stent according to claim 4 and wherein said originally flat, pattern is formed from a piece of flat metal.
